# EUROPEAN PATENT SPECIFICATION

(11) **EP 3 612 618 B1**
(45) Date of publication and mention of the grant of the patent: **10.03.2021**
(21) Application number: 18717392.7
(22) Date of filing: 20.04.2018
(51) Int. Cl.: C10G 3/00, C10L 1/02

(54) **FUEL COMPOSITIONS COMPRISING C3 ALCOHOLS**
BRENNSTOFFZUSAMMENSETZUNGEN MIT C3-ALKOHOLEN
COMPOSITIONS DE COMBUSTIBLE COMPRENANT DES ALCOOLS EN C3

(30) Priority: 21.04.2017 IT 201700044195
(43) Date of publication of application: 26.02.2020
(73) Proprietor: ENI S.p.A., 00144 Roma (IT)
(72) Inventor: REBESCO, Elena Maria, 20132 Milano (IT); CALEMMA, Vincenzo, 20097 San Donato Milanese (MI) (IT)
(74) Representative: Cernuzzi, Daniele
(86) International application number: PCT/EP2018/060144
(87) International publication number: WO 2018/193074

(56) References cited:
- WO-A2-2014/080379
- US-A- 4 743 272
- US-A1- 2013 291 429
- US-A1- 2016 108 330

## Description

### FIELD OF APPLICATION

The present invention relates to the fuel sector and in particular the bio-fuel sector, i.e. fuels of biological origin or of fossil origin but comprising components of biological origin. In particular, the present invention relates to a composition comprising a hydrocarbon mixture that can be used as fuel, propanol and propanediol in appropriate proportions. The aforementioned composition can be advantageously used as fuel for Otto engines and gasoline engines in general. In particular, when propanol and/or propanediol are obtained starting from renewable biological sources, the composition according to the present invention can be considered an "advanced" biofuel, as better specified below.

### Prior art

It is known that the emissions produced by burning fuels of fossil origin containing carbon dioxide (CO₂), carbon monoxide (CO), nitrogen oxides (NOₓ), sulfur oxides (SOₓ), unburned hydrocarbons (HC), volatile organic compounds and particulate matter (PM), are the cause of environmental problems, such as the production of ozone, the greenhouse effect (in the case of nitrogen and carbon oxides), acid rain (in the case of sulfur and nitrogen oxides).

The constant increase in the consumption of fuels for transport, and the increasingly greater sensitivity towards the environment, along with an increasingly stringent international framework of legislation in relation to pollutant emissions and greenhouse gases, have led to the constantly greater importance of processes to allow fuels to be obtained from renewable sources, so-called bio-fuels.

In particular, with the adhesion to the Kyoto Protocol, the European Union has issued a series of directives, known as the "20/20/20 *package",* which set out the aim of reducing global warming due to human activity.

Within the context of the transport sector the RED (Renewable Energy Directive) 2009/28/EC and subsequent amendments, imposes the addition of bio-components to fossil fuels in increasing quantities until reaching 10% of the total energy composition, by the year 2020.

The Fuel Quality Directive, approved in 2012, confirmed the objective of 10% of energy produced from bio-fuels, also allowing the contribution of bio-fuels from second generation renewable sources, which do not compete with the food chain (used food oils, animal fats, tall oil, etc.), to be considered as having double value. Finally, the revision of the RED Directive, known as the "ILUC Directive" set a 7% limit for bio-fuels deriving from food crops, encouraging the use of "advanced" bio-fuels (i.e. deriving from municipal waste, algae, waste effluents containing crude glycerin, lignocellulosic biomass, etc.), which by 2020 must be used at least in the proportion of 0.5%.

The need to produce fuels from renewable sources, in order to comply with the aforementioned European directives, has led to the development of processes for obtaining components of bio-fuels that use as starting fillers products of biological origin derived from the conversion of lignocellulosic biomass or from the production of oils and fats of animal or plant origin, or mixtures of said products.

An oxygenated compound, which can also be obtained from renewable sources, commonly added to fuels, is ethanol. It is produced through fermentation processes starting from different materials such as sugar cane, sugar beet; grains such as corn, barley, potatoes, cassava; lignocellulosic biomass such as agricultural waste, straw and forest residues.

Ethanol displays a series of positive characteristics, including its high octane number, and a density similar to that of gasoline. The use of gasolines containing ethanol in spark ignition engines can lead to a substantial reduction in the emission of pollutants generated by combustion, as described for example by U. Arjun et al., in "Ethanol Gasoline Blends in SI Engine" (2014), International Journal of Scientific and Engineering Research, vol. 5, pag. 881-886. The presence of an oxygenated molecule in the fuel tends to increase the air/hydrocarbon ratio and promote the combustion of the fuel itself. Furthermore, the oxygen of ethanol promotes a reduction of the emission of carbon monoxide and of volatile organic compounds (VOCs).

In general, the quantity of ethanol added to the hydrocarbon mixture can vary from 5% by volume (in so-called E5 gasolines) up to a maximum of 85% (in E85 gasolines). The most common mixtures contain ethanol in an approximate percentage of 10% (E10) and 25% (E25). In particular, gasolines with a lower ethanol content such as E5, E7 and E10, can be used in internal combustion engines for vehicles without any mechanical adjustments to the engine or to the fuel supply system.

In Europe, in accordance with the specific European standard EN228:2013, which provides the chemical/physical characteristics of unleaded gasolines sold in the states of the European Union and the analytical methods for determining such characteristics, unleaded gasoline may contain up to 5.0% by volume of ethanol or bio-ethanol, i.e. ethanol from renewable sources, (called E5) or up to 10.0% by volume of ethanol or bio-ethanol, i.e. ethanol from renewable sources (called E10). The ethanol must comply with the EN 15376 standard. The use of ethanol mixed with gasoline is not free from drawbacks. In fact, ethanol is hygroscopic, miscible with water and immiscible with hydrocarbon mixtures in a wide temperature range. This leads to demixing with hydrocarbons and consequent phase separation as described, for example, by Zlata Mužiková, et al. in "Volatility and phase stability of petrol blends with ethanol" (2009), Fuel, vol. 88, pag. 1351-1356. In general, to contrast demixing, gasolines containing ethanol are stabilized with additives such as MTBE.

Furthermore, ethanol is characterized by low calorific power, and the high latent heat of vaporization can cause problems for cold starts; additionally, ethanol can form azeotropes with light hydrocarbons causing an increase in the volatility of the fuel containing it.

Furthermore, in cases in which ethanol is contained in percentages higher than 5% by volume, gasoline mixtures with ethanol are characterized by higher volatility values (expressed, for example as, the Reid Vapor Pressure, RVP, i.e. the vapor pressure exerted by a liquid at 37.8°C as determined through the standard method ASTM D323-15a, or as the Dry Vapor Pressure Equivalent, DVPE, as determined through the standard method ASTM D5191-15). This negative effect on the volatility of the hydrocarbon mixture obtained can be mitigated or neutralized by changing the composition of the starting gasoline, for example, by varying the content of aromatic hydrocarbons, butane, total olefins. In other words, special care must be taken in the choice of starting gasoline so as to obtain hydrocarbon mixtures in which the vapor pressure and the other parameters connected thereto, fall within well-defined limits, in order to fulfil the specifications, as described, for example, in US 4,207,077.

It is important to note that an increase in vapor pressure and therefore in the volatility of the gasoline is considered disadvantageous as it promotes increased emissions of volatile organic compounds (so-called VOCs) that contribute to the formation of smog. The aforementioned specification EN228 suggests the use of alternative oxygenated compounds to ethanol, in particular alcohols having from 1 to 4 carbon atoms or ethers having a number of carbon atoms greater than or equal to 5.

Based on this specification, as an alternative to ethanol, it is possible to use iso-butanol (up to 15% by volume) or t-butyl-alcohol (up to 15% by volume) as components to be added to fuels; the latter have better miscibility with hydrocarbons with respect to that of ethanol. However, at low temperatures even butanol-gasoline mixtures remain inhomogeneous.

Propanol also has the same positive characteristics as ethanol with reference to its ignition qualities and calorific power, but it has a higher energy density (+12%), lower volatility, and lower latent heat of vaporization (-8%).

The use of P20 mixtures (20% by volume of propanol and 80% by volume of gasoline) are described for example by B. M. Masum et al., in "Performance and emission analysis of a multi cylinder gasoline engine operating at different alcohol-gasoline blends" (2014) Royal Society of Chemistry Advances, vol. 4, pag. 27898-27904. In particular, the author compares the thermal efficiency of mixtures of gasoline with methanol, ethanol, propanol, or butanol, concluding that propanol and butanol represent the most effective options for the formulation of fuel compositions with respect to ethanol in terms of the properties of the compositions, engine performance and fuel emissions.

The possible additives allowed by European specification EN 228 also include methanol, up to 3%. This additive has solvent properties, increases the anti-knock power, has lower calorific power (equal to about 51%) with respect to gasoline, but requires less comburent.

However, as already described in the case of ethanol, gasoline and methanol mixtures can be unstable and, particularly when the concentration of this alcohol exceeds the value indicated in the specification, it can cause the demixing of the composition with phase separation, and increase its volatility.

The Applicant therefore set out to identify a new and more advantageous composition comprising gasoline mixed with alcohols having a number of carbon atoms comprised between 1 and 4, whose concentration is not necessarily constrained to the limits over which the aforementioned negative effects arise, and that therefore has better performance levels as a fuel than the compositions described in the prior art, in full compliance with legislation on emissions.

Furthermore, the Applicant set out to prepare the aforementioned composition at least partially from compounds deriving from renewable fuels through simple and cost-effective processes, in which the purification steps are reduced to a minimum.

In particular, the Applicant set out to prepare the aforementioned composition starting from glycerin of biological origin, whose market towards traditional sectors such as the cosmetic and pharmaceutical sector, and the production of acrylic derivatives, is currently saturated. US 2013/291429 and US 2016/108330 both disclose fuel compositions.

### Description of the invention

These and other objectives are now surprisingly reached with the composition in accordance with the present invention and the preparation method thereof. The present invention therefore relates to a fuel composition for gasoline engines, comprising at least one gasoline blend, propanol and 1,2-propanediol, and optionally one or more alcohols different from those mentioned, having a number of carbon atoms comprised between 1 and 4. The fuel composition of the invention and the preparation process of the invention are defined in the appended claims.

In particular, the aforementioned fuel composition comprises from 0.2% to 20% by volume of propanol and from 0.1 % to 10 % by volume of 1,2-propanediol, with respect to the total volume of the fuel composition, wherein said propanol and 1,2-propanediol are respectively present in quantities such that their ratio by volume is greater than 2 and preferably comprised between 2 and 50.

Optionally, the fuel composition in accordance with the invention may further contain one or more other alcohols having a number of carbon atoms comprised between 1 and 4, particularly methanol, ethanol and any one of the isomers of butanol, in a concentration comprised overall between 0.1% by volume and 10% by volume with respect to the total volume of the composition.

According to a particular aspect of the present invention, said fuel composition may comprise a relatively low amount of water, preferably in an amount lower than 10,000 ppm, more preferably lower than 5,000 ppm, even more preferably in the range from 50 to 1,000 ppm, by wt. with respect to the total weight of the composition.

The use of a mixture of propanol and 1,2-propanediol in the aforementioned concentrations, displays a co-solvent property of methanol and ethanol, by stabilizing the phases and reducing the vapor pressure of the fuel composition obtained, so as to fulfil ASTM specifications and the requirements dictated by European and international legislation.

Therefore, the composition of the present invention is characterized by the fact that it may contain, for the same amount of other desirable fuel properties, higher concentrations of oxygenated compounds, and in particular higher concentrations of bonded oxygen, able to produce a relatively lower quantity of gaseous pollutants.

Furthermore, the composition according to the present invention has the advantage of allowing a reduction in the MTBE content as an additive and preferably the aforementioned composition allows a substantially null quantity of MTBE to be used. The fuel composition in accordance with the invention may be advantageously used as a fuel for Otto engines and in general for gasoline engines, allowing during combustion a reduction of the emission of harmful substances such as CO, NOₓ and VOC to be obtained.

Further characteristics and advantages of the present invention will become clear from the following detailed description.

The present invention further relates to a method for preparing the aforementioned fuel composition, said method comprising the following steps:
a) hydrogenation of glycerin to obtain a mixture comprising water, propanol and 1,2-propanediol, wherein propanol and 1,2-propanediol are in such proportions that their volume ratio is comprised between 2 and 50;
b) separating water and at least one part of any unreacted glycerin from said first mixture derived in step (a), to obtain a second mixture comprising propanol and 1,2-propanediol; and
c) adding said mixture to a gasoline, preferably of fossil origin, in amounts comprised between 0.2% and 40%, preferably between 1% and 30% by volume, relative to the volume of the resulting mixture, to obtain a fuel composition according to the invention.

Other objects of the present invention shall be made apparent below in the present description and claims.

For the purposes of the present description and following claims, the definitions of the numeric ranges always include the extremes unless specified otherwise.

For the purpose of the present description and following claims, the percentages are always by volume, except in cases in which it is specified otherwise. The percentages by volume refer to ideal mixtures that comply with the rule of additivity of volumes. In the mixtures and compositions in accordance with the present invention, deviations from ideality are not observed.

In the description of the embodiments of the present invention, the use of the terms "comprising" and "containing" indicates that the options described, for example regarding the steps of a method or of a process or the components of a product or of a device, are not necessarily all-inclusive. It is however important to note that the present application also relates to the embodiments in which the term "comprising" in relation to the options described, e.g. regarding the steps of a method or of a process or the components of a product or of a device, must be interpreted as "which essentially consists of" or "which consists of", even if this is not explicitly stated.

For the purposes of the present description and following claims, the terms "gasoline" or "gasoline blend" mean a mixture prevalently comprising hydrocarbons such as, by way of example, paraffins, aromatic hydrocarbons, olefins and naphthenes, typically having from 3 to 12 carbon atoms, which can be used as fuel, characterized by an End Point (ASTM D86) not greater than 250 °C, preferably not greater than 210 °C, where the End Point means the temperature at which 100 % by volume of said hydrocarbon mixture is distilled. Said gasoline may have a density comprised between 700 and 800, preferably between 720 and 775, kg/m³. Usable gasolines are those deriving from catalytic processes, preferably deriving from Fluid Catalytic Cracking (FCC) processes, from reforming processes, and mixtures thereof, according to what is generally known in the art. Preferably the sulfur content of these gasoline blends is comprised between 50 and 0.1 mg/kg, and even more preferably between 10 and 0.5 mg/kg. Unleaded gasolines are particularly preferred, which comprise mixtures of hydrocarbons having boiling points at atmospheric pressure in a relatively narrow temperature range, for example comprised between 25°C and 225°C. Some gasolines may contain oxygenated compounds, such as alcohols (e.g., ethanol, propanol), or ethers (e.g., methyl-t-butyl-ether, MTBE). The gasolines may also comprise different additives such as detergents, anti-freeze agents, emulsion breakers, corrosion inhibitors, dyes, anti-depositing agents and octane boosters.

For the purposes of the present description and following claims, "from renewable sources" (e.g. "glycerin from renewable sources") means compounds not obtained from fossil resources, such as crude oil, carbon, natural gas, oil sands, etc., but directly from plant biomass, algae, microorganisms or from the treatment of more complex compounds derived from said plant biomass, algae and microorganisms.

For the purposes of the present description and following claims, the term propanol, unless specified otherwise, means overall the set of isomers of propanol present in the fuel composition as claimed, i.e. 1-propanol, 2-propanol or both the isomers in mixture in any proportion with each other.

The present invention relates in particular to a composition useful as a fuel for gasoline engines, comprising:
- from 60.0% to 99.7%, preferably from 80% to 90%, of a hydrocarbon mixture corresponding to a gasoline blend,
- from 0.2 to 20%, preferably from 1% to 15%, of propanol
- from 0.1 to 10%, preferably from 0.5% to 5%, of 1,2-propanediol, and
- optionally from 0% to 20%, preferably from 0% to 10%, of one or more aliphatic alcohols, other than propanol and 1,2-propanediol, having a number of carbon atoms comprised between 1 and 4,
wherein said propanol and 1,2-propanediol are in such amounts that their volume ratio is comprised between 2 and 50, preferably between 3 and 20.

In a preferred aspect of the invention, said composition useful as a fuel for gasoline engines, may comprise at least one alcohol selected from methanol, ethanol, 1,3-propanediol, in particular methanol. In that case, said at least one alcohol is preferably in an amount from 1% to 3% by volume, even more preferably from 1.5% to 2% by volume, relative to the total volume of the composition.

The composition of the present invention is prepared through mixing the individual components or mixtures of the individual components. Any other additives present in the final composition may be introduced either to the final composition itself or by mixing with one or more of the individual components prior to the preparation of the final composition.

In a preferred aspect of the present invention, the fuel composition in accordance with the present invention is prepared by adding to the gasoline blend a mixture comprising propanol and 1,2-propanediol, wherein the relative concentration of 1,2-propanediol is comprised between 2% and 50%, more preferably between 5% and 30% by volume with respect to the volume of propanol.

According to a preferred embodiment of the present invention, said propanol is constituted by 0 to 10% of 2-propanol, the remaining part being 1-propanol.

According to a different embodiment of the present invention, said propanol is constituted by 10 to 30% of 1-propanol, the remaining part being 2-propanol.

According to a further embodiment of the present invention, said propanol in the fuel composition is constituted by 10.1% to 50% of 2-propanol, the remaining part being 1-propanol.

In accordance with the present invention, no substantial differences have been noted in the properties of the fuel composition when the propanol component is constituted only by 1-propanol, only 2-propanol or a mixture of the aforesaid.

According to a particular aspect, said mixture of propanol and 1,2-propanediol mixed with the gasoline blend in the composition according to the present invention also comprises between 0.1% and 10% by volume of 1,3-propanediol, with respect to the total volume of said mixture.

Hydrocarbon mixtures corresponding to the gasoline blend characterized by an End Point not exceeding 250°C, preferably not more than 210°C, having a density comprised between 720 and 775 kg/m³ are advantageously usable in the composition and in the process according to the present invention.

Advantageously usable hydrocarbon mixtures are those deriving from catalytic processes, preferably from Fluid Catalytic Cracking (FCC) processes, reforming processes, and mixtures thereof.

Preferably the sulfur content of these hydrocarbon mixtures is comprised between 1 mg/kg and 2000 mg/kg, and even more preferably comprised between 1 mg/kg and 10 mg/kg.

According to a particularly preferred aspect of the present invention, said fuel composition is prepared starting from glycerin, preferably of biological origin, and a gasoline blend, preferably an unleaded gasoline and with low sulfur content, through a process comprising the following steps:
a) hydrogenation of glycerin to obtain a first mixture comprising water, propanol and 1,2-propanediol, wherein propanol and 1,2-propanediol are in such proportions that their volume ratio is comprised between 2 and 50;
b) separating water and at least one part of any unreacted glycerin from said first mixture derived in step (a), to obtain a second mixture comprising propanol and 1,2-propanediol; and
c) adding said second mixture to a gasoline blend, preferably of fossil origin, in amounts comprised between 0.2% and 40%, preferably between 1 and 30 % by volume, relative to the resulting volume of the mixture, to obtain a fuel composition according to the invention.

Step (a) of said process can be performed starting from glycerin according to any one of the known methods for the reduction of hydroxyl groups.

Hydrogenation catalysts advantageously usable are, for example, copper chromite, mixed oxides of chromium-zinc-copper, noble metals supported on coal, noble metals supported on iron oxide, and preferably they are palladium supported on coal, platinum supported on coal and palladium supported on iron oxide, the latter catalyst being the most preferred.

The hydrogenation reaction of glycerin may be performed at a temperature comprised between 100°C and 400°C, under hydrogen pressure comprised between 0.1 MPa and 10 MPa.

The process conditions and catalysts suitable for the hydrogenation reaction of glycerin are described in literature, for example by Z. Xiao et al. in "Insight into the reaction pathways of glycerol hydrogenolysis over Cu-Cr-catalysts" (2012) J. Mol. Catal. To: Chemical, vol. 365, pag. 24-31, or by M.G. Musolino et al., in "Selective transfer hydrogenolysis of glycerol promoted by palladium catalysts in absence of hydrogen" (2009), Green Chemistry, vol. 111, pag. 1511 -1513.

The hydrogenation of glycerin can be appropriately controlled with the aim of obtaining, at the end of the reaction, either at least the reduction intermediate 1,2-propanediol, or at least propanol in the desired proportions.

For example, patent application DE102008026583 describes a process for obtaining mixtures of 1 -propanol and/or 2-propanol and/or ethanol that comprises a first catalytic hydrogenation step of glycerin for obtaining 1,2-propanediol and therefore a second catalytic hydrogenation step of said 1,2-propanediol for obtaining 1-propanol, possibly in mixture with 2-propanol and/or ethanol. It is therefore clear that by varying the conditions described in DE102008026583 it is possible to obtain a mixture comprising both the intermediate 1,2-propanediol and propanol in the desired proportions.

Depending on the catalyst used and the reaction conditions, it is also known that it is possible to obtain as the final reduction product 1-propanol or 2-propanol or either 1-propanol or 2-propanol in a mixture with each other and with 1,2-propanediol in the desired proportions. In particular, for example, it is possible to select the process conditions so that the mixture obtained at the end of step (a) comprises 2-propanol and 1-propanol in a molar ratio to each other comprised between 0 and 0.1, or so that the mixture obtained at the end of step (a) comprises 1-propanol and 2-propanol in a molar ratio to each other comprised between 0.1 and 0.5.

Preferably, the hydrogenation of glycerin for obtaining a first mixture comprising propanol and 1,2-propanediol in the desired proportions, is performed in a single reaction step, or in two sub-steps in sequence, first substantially producing 1,2-propanediol and then by hydrogenating part of this to propanol. In the event that the hydrogenation is performed in two sub-steps, they may be performed in two separate reactors or in adjacent zones of the same reactor, in which there are specific conditions of temperature, hydrogen pressure and type of catalyst.

Preferably, the conversion of glycerin is at least 50%, more preferably from 55% to 99%, even more preferably from 60% to 90%.

In the hydrogenation conditions described above, mixtures of propanol and 1,2-propanediol containing lower quantities of 1,3-propanediol can be obtained, preferably up to 5% in moles with respect to the moles of propanediol respectively produced. Alcohols other than glycerin may also be present, such as ethanol, methanol and ethylene glycol. In accordance with the present invention, the glycerin is fed in step (a) substantially pure, preferably with purity from 98.0 % to 99.9 %. Such degree of purity can be obtained from crude glycerin, particularly that coming from biodiesel production processes, through purification and whitening processes well known in the art.

In accordance with step (b) of the process according to the present invention, said first mixture comprising propanol and 1,2-propanediol obtained from the hydrogenation of glycerin in step (a), which normally also contains unreacted glycerin, any acetol obtained as an intermediate sub-product during the hydrogenation step, and water, present as a possible solvent and as a reaction product, can be separated from its main components according to distillation methods known in the art. In fact, the chemical/physical characteristics of each of the components and of their mixtures, and the related liquid-vapor diagrams, are well known and reported in literature. Propanol and 1,2-propanediol, together with any isomers formed, can then be added to the gasoline blend, as such or mixed together, in accordance with step (c) of the process.

According to a preferred aspect of the present invention, the mixture of propanol and 1,2-propanediol, with any other C1-C3 alcohols different from glycerin that may have been formed in the reaction in a total amount not exceeding 10%, is separated as such in step (b) of the present process, and used directly or after any drying step for eliminating any residual humidity still present, for the formation of the fuel composition in accordance with the present invention. The separation of the mixture is normally obtained by distillation, according to known methods.

Preferably, in step (b) all the water present is substantially separated, except for traces of humidity compatible with the subsequent addition to the gasoline blend. Preferably, said second mixture obtained in step (b) contains from 0.1 to 1%, more preferably from 0.1 to 0.5%, by volume of residual water. Unreacted glycerin and any acetol are preferably separated almost completely in step (b), to obtain said second mixture substantially comprising 1-propanol, 1,2-propanediol and any C1-C4 alcohols other than glycerin. However, in accordance with the present invention, a modest quantity of glycerin is tolerable, generally less than 2% by volume.

Step (c) of the present process may be performed by simply mixing 1-propanol and 1,2-propanediol, or preferably their mixture, as obtained in step (b), and the desired gasoline blend, as appropriately selected by a person skilled in the art, in the desired amounts, within the claimed range. The order of addition or mixing is not particularly significant. Where considered appropriate, a person skilled in the art may however mix the components in subsequent portions or by the continued addition of one component to the other, until the final composition. In general, it is preferable to add the mixture of 1-propanol and 1,2-propanediol to the gasoline blend.

For the purpose of maximizing the consumption of glycerin and obtaining a fuel composition with optimal properties, it is preferable to add an amount of the 1-propanol and 1,2-propanediol mixture, comprised between 2 and 15% by volume with respect to the volume of the resulting composition, to the gasoline blend.

A mixture of aliphatic alcohols, having a number of carbon atoms comprised between 1 and 4, other than propanol and propanediols may optionally be added to the hydrocarbon mixture corresponding to a gasoline blend, mixed with appropriate quantities of propanol and 1,2-propanediol, preferably comprised in the mixture obtained according to the aforementioned hydrogenation step.

When present, said one or more aliphatic alcohols having a number of carbon atoms comprised between 1 and 4, other than propanol and propanediol are comprised in a relative concentration comprised between 0.1% and 5% by volume with respect to the volume of the composition, and preferably from 1% to 3% by volume, even more preferably from 1.5% to 2% by volume, with respect to the total volume of the composition.

Specific examples of aliphatic alcohols, other than propanol and 1,2-propanediol, having numbers of carbon atoms comprised between 1 and 4, particularly useful for the purpose of the present invention are: methanol, ethanol, 1-butanol, 2-butanol, t-butyl-alcohol. Preferably methanol and ethanol.

Preferably, said aliphatic alcohols, especially ethanol, are obtained biologically, i.e. for example, by the fermentation of biomass or of biomass derivatives, i.e. by the fermentation of biomass deriving from agricultural crops rich in carbohydrates and sugars, or by the fermentation of lignocellulosic biomass, or by the fermentation of algal biomass.

The aforementioned aliphatic alcohols may also derive for example from the fermentation of at least one biomass of microorganisms or algae or micro-algae, or by the fermentation of residues or derivatives from the crop of said biomass.

In a preferred aspect of the invention, when the composition of the present invention comprises two or more aliphatic alcohols, other than 1-propanol and 1,2-propanediol, having a number of carbon atoms comprised between 1 and 4, at least one of said aliphatic alcohols is selected from methanol and ethanol and more preferably it is methanol.

Therefore, in a preferred aspect of the invention, said composition comprises at least one aliphatic alcohol other than propanol and 1,2-propanediol, where said aliphatic alcohol is methanol in a concentration comprised between 0.1% and 5% by volume, more preferably between 1% and 3% by volume, with respect to the total volume of the composition. Said methanol is preferably added to the fuel composition in step (c) of the process according to the present invention.

The composition according to the present invention may possibly comprise conventional additives known in the state of the art, such as octane boosters, anti-freeze, emulsion breakers, detergents, antioxidants, corrosion inhibitors, anti-static additives, dyes, anti-deposition agents or mixtures thereof. Generally, said additives are possibly present in an amount not exceeding 0.3% by volume relative to the total volume of said composition. The composition useful as a fuel for gasoline engines according to the present invention is therefore characterized in that it comprises, as oxygenated components, both propanol and 1,2-propanediol, and optionally aliphatic alcohols, other than the previous ones, having a number of carbon atoms comprised between 1 and 4. Propanol and 1,2-propanediol represent components for bio-fuels of the so-called "advanced" type. Furthermore, unlike other alcohols normally used as blending components, such as methanol or ethanol, they contribute to reducing the vapor pressure of the resulting hydrocarbon composition and display, in the proportions as claimed, excellent co-solvent power towards methanol, much better than has been observed with ethanol in corresponding compositions.

It is precisely this characteristic that allows aliphatic alcohols such as methanol or ethanol to be added to aforementioned composition, while maintaining the final composition within the specifications envisaged by European and international standards.

### EXAMPLES

For the purpose of putting the present invention into practice and illustrating it more clearly, below are some non-limitative examples. The following methods were applied for measuring the properties of the fuel compositions:
Distillation curve (according to standard ASTM D86), hence revealing the sample percentage volume that evaporates at 70°C (called E70);
- Vapor pressure (VPR), determined according to method ASTM D5191, measured in kPa;
- Octane number (RON), determined according to method ASTM D2699.

In the examples, the following commercial products were used for the formation of the fuel compositions:
- 1-propanol (Sigma Aldrich; purity ≥ 99.5 %)
- 2-propanol (Carlo Erba; purity ≥ 99.9 %)
- 1,2-propanediol (Sigma Aldrich; purity = 99%)

### EXAMPLE 1: Production of "advanced" gasoline directly from glycerin.

A fuel composition in accordance with the present invention was prepared through the reduction of glycerin with hydrogen to obtain a mixture prevalently containing 1-propanol and 1,2-propanediol, and subsequently adding such mixture to a gasoline blend of fossil origin coming from a refinery. 100 ml of a solution comprising 60% by volume of glycerin and 40% by volume of water, were poured into a steel autoclave having a volume of 200 ml and provided with a mechanical stirrer. 6.5 g of hydrogenation catalyst comprising copper chromite were then added. The hydrogenation reaction was performed at the temperature of 240°C, with a residence time of 300 minutes and a hydrogen pressure of 6 MPa. After the reaction time, the autoclave was cooled, depressurized and the reaction products were analyzed through gas chromatography. The results of the analysis of the reaction mixture highlighted:
Glycerin conversion 58%;
Molar selectivity to 1-propanol: 61%;
Molar selectivity to 1,2-propanediol: 29%;
Molar selectivity to acetol: 6%;
Molar selectivity to 2-propanol: 3%

The complement to 100 of the selectivity is to be attributed to the presence of sub-products in traces such as ethyl alcohol, propane, ethane, ethylene glycol, 1,3-propanediol.

From the reaction mixture thus obtained, a mixture substantially containing 1-propanol, 1,2-propanediol and 2-propanol, in the aforementioned proportions, is separated by distillation according to the known techniques, which is added to a gasoline of fossil origin having the characteristics indicated in Table 1 below, in proportions (alcohol mixture)/(gasoline) of 10/90 by volume. The components are mixed at the temperature of about 5°C. After stirring for about 2 minutes, a homogeneous mixture is obtained, usable as fuel.

**TABLE 1: characteristics of a refinery gasoline**

| Properties | Values |
|---|---|
| Density (kg/m³) | 748.3 |
| VPR (kPa) | 52.3 |
| RON | 94.3 |
| Aromatics (%vol) | 32.1 |
| Olefins (%vol) | 10.3 |
| Distillation curve | |
| Start point (°C) | 35.0 |
| Distilled to 50% (°C) | 104.5 |
| End point (°C) | 210.0 |
| Evaporated at 70°C (%vol) | 24.3 |
| Evaporated at 100°C (%vol) | 47.9 |
| Evaporated at 150°C (%vol) | 83.2 |

### EXAMPLE 2: gasoline containing propanol and 1,2-propanediol

For the purpose of highlighting the advantageous properties of the compositions according to the present invention, some model mixtures were prepared by adding predetermined quantities of alcohols and diols, as such or mixed together, according to the indications of Table 2, to gasoline of fossil origin from a refinery having the properties indicated in Table 1. Five compositions were prepared, three of which being comparative (Comp. test 1, 2 and 3) and two according to the invention (Test 1 and Test 2).

The mixtures, having a volume of about one liter, were prepared by mixing the components at about 5°C through mechanical agitation for about 2 minutes, in a 2.5 liter refrigerated glass container, then left to rest for at least 1 hour.

The following properties were measured on the compositions thus obtained: E70, VPR and RON, the results of which are shown in Table 2.

**Table 2: gasoline compositions with alcohols and diols**

| Component/ property | Gasoline as such | Comp. test 1 | Comp. test 2 | Comp. test 3 | Test 1 | Test 2 |
|---|---|---|---|---|---|---|
| 1 -propanol (%vol) | 0 | 10 | 9 | 0 | 8.1 | 6.65 |
| 2-propanol (%vol) | 0 | 0 | 1 | 0 | 0.4 | 0.35 |
| 1,2-propanediol (%vol) | 0 | 0 | 0 | 10 | 1.5 | 3 |
| E70 (%vol) | 24.3 | 26.4 | 27.5 | N.A. (¹) | 26.3 | 26.8 |
| VPR (kPa) | 52.3 | 52.8 | 52.7 | N.A. (¹) | 52.5 | 52.5 |
| RON | 94.3 | 95.9 | 96.0 | N.A. (¹) | 96.0 | 96.1 |

| | | | | | | |
|---|---|---|---|---|---|---|
| (¹) Not miscible | | | | | | |

From the comparison of the data indicated in Table 2, it is highlighted that, unlike what happens when it is introduced alone into gasoline, 1,2-propanediol mixed with propanol (either normal or iso), mixes with the hydrocarbon gasoline. It is therefore possible to use mixtures of propanol/1,2-propanediol instead of using pure propanol.

### EXAMPLE 3: Compositions containing methanol, propanol and 1,2-propanediol

Predetermined quantities of alcohols and diols, as such or mixed together, according to the indications of Table 3, were added to a gasoline of fossil origin coming from a refinery, having the properties indicated in Table 1. Three compositions were prepared, two of which being comparative (Comp. test 4 and 5) and one according to the invention (Test 3).

The mixtures were prepared in the laboratory, mixing the components at about 5°C. The following properties were measured on the fuel compositions thus prepared: E70, VPR and RON, the results of which are shown in Table 3.

**Table 3: gasolines with ternary mixtures of 1-propanol_{.} 2-propanol and 1,2-propanediol in the presence of methanol and ethanol.**

| Properties/Contents | Comp. test 4 | Test 3 | Comp. test 5 |
|---|---|---|---|
| Methanol (%vol) | 2 | 2 | 2 |
| 1 -propanol (%vol) | 1.9 | 1.62 | 0 |
| 2-propanol (%vol) | 0.1 | 0.08 | 0 |
| 1,2-propanediol (%vol) | 0 | 0.3 | 0 |
| Ethanol (%vol) | 0 | 0 | 2 |
| E70 (%vol) | 29.4 | 29.7 | 33.2 |
| VPR (kPa) | 66.8 | 66.5 | 71.4 |
| RON | 94.9 | 95.2 | 94.5 |

In the presence of methanol, the vapor pressure of gasolines containing propanol and propanol/1,2-propanediol is lower than the vapor pressure of gasoline containing ethanol. Propanol and 1,2-propanediol in combination with each other are therefore good cosolvents of methanol.

The presence of 1,2-propanediol (test 6) does not alter the good co-solvency characteristic of propanol alone (case 1), therefore it is possible to use mixtures of propanol/1,2-propanediol, possibly obtained as such by the direct hydrogenation of glycerin, instead of using pure propanol.

Finally, it is however to be understood that further changes and variations may be made to the process and compositions described and illustrated herein which do not depart from the scope of the appended claims.

## Claims

1. A useful composition as a fuel for gasoline engines comprising a hydrocarbon mixture corresponding to a gasoline blend, propanol, 1,2-propandiol and optionally one or more aliphatic alcohols, other than propanol and 1,2-propanediol, having a number of carbon atoms comprised between 1 and 4, said composition comprising:
- from 60.0% to 99.7% of a hydrocarbon mixture corresponding to a gasoline blend,
- from 0.2 to 20% of propanol,
- from 0.1 to 10% of 1,2-propanediol, and
- optionally from 0% to 20% of one or more aliphatic alcohols, other than propanol and 1,2-propanediol, having a number of carbon atoms comprised between 1 and 4
wherein said propanol and 1,2-propanediol are respectively in such amounts that their volume ratio is comprised between 2 and 50, and wherein said gasoline blend is **characterized by** an End Point not exceeding 250°C and has a density between 720 and 775 kg/m³ .

2. Composition according to claim 1, comprising:
• from 80% to 94% of a hydrocarbon mixture corresponding to a gasoline blend,
• from 1% to 15% of propanol,
• from 0.5% to 5% of 1,2-propanediol, and
• optionally from 0% to 10% of one or more aliphatic alcohols, other than propanol and 1,2-propanediol, having a number of carbon atoms comprised between 1 and 4,
wherein said propanol and 1,2-propanediol are respectively in such amounts that their volume ratio is comprised between 3 and 10.

3. Composition according to claim 1 or 2, wherein said aliphatic alcohol, other than propanol and 1,2-propanediol, is selected from ethanol, methanol or a mixture of the aforesaid.

4. Composition according to any one of the preceding claims, comprising methanol in an amount from 0.1% to 5%, preferably from 1% to 3%, more preferably from 1.5% to 2% by volume, relative to the total volume of the composition.

5. Composition according to any one of the preceding claims, wherein said propanol is constituted by 0 to 10% of 2-propanol, the remaining part being 1-propanol.

6. Composition according to any one of the preceding claims, wherein said propanol is made up of 10 to 30% of 1-propanol, the remaining part being 2-propanol.

7. Composition according to any one of the preceding claims, wherein said propanol is constituted from 10.1% to 50% of 2-propanol, the remaining part being 1-propanol.

8. Composition according to any one of the preceding claims, wherein said gasoline blend is **characterized by** an End Point not exceeding 210°C.

9. Composition according to any one of the preceding claims, comprising water in an amount lower than 10,000 ppm, preferably lower than 5,000 ppm, more preferably in the range from 50 to 1,000 ppm, by wt. with respect to the total weight of the composition.

10. Process for the preparation of a fuel composition according to any one of the preceding claims from 1 to 9, starting from glycerin, preferably of biological origin, and a gasoline blend, preferably unleaded gasoline and with a low sulfur content, said process comprising the following steps:
a) hydrogenation of glycerin to obtain a first mixture comprising water, propanol and 1,2-propanediol, wherein said propanol and 1,2-propanediol are in such proportions that their volume ratio is comprised between 2 and 50, preferably between 3 and 10;
b) separation of water and of at least one part of any unreacted glycerin from said first mixture derived in step (a), to obtain a second mixture comprising propanol and 1,2-propanediol; and
c) addition of said second mixture to a gasoline blend, preferably of fossil origin, in amounts comprised between 0.2% and 40%, preferably between 1 and 30% by volume, relative to the volume of the resulting mixture, to obtain a fuel composition.

11. Process according to claim 10, wherein in said step (a) the conversion of glycerin is at least 50%, more preferably from 55% to 99%, still more preferably from 60% to 90%.

12. Process according to any one of the preceding claims 10 or 11, wherein said second mixture obtained in step (b) is substantially constituted by propanol, 1,2-propanediol and up to a maximum of 10% by volume of C1 -C3 alcohols other than glycerin.

13. Process according to any one of the preceding claims 10 to 12, wherein said propanol in the second mixture obtained in step (b) comprises from 0 to 10% 2-propanol, the remaining part being 1 -propanol.

14. Process according to any one of the preceding claims 10 to 13, wherein said hydrogenation step (a) is conducted in the presence of a hydrogenation catalyst at a temperature comprised between 100°C and 400°C, under hydrogen pressure comprised between 0.1 MPa and 10 MPa.

15. Process according to any one of the preceding claims 10 to 14, wherein in said step (c) methanol is also added to the gasoline blend in an amount comprised between 0.1% and 5% by volume, more preferably between 1% and 3% by volume, relative to the total volume of the composition.

## Patentansprüche

1. Nutzzusammensetzung als ein Kraftstoff für Benzinmotoren, umfassend ein Kohlenwasserstoffgemisch, das einem Benzingemisch entspricht, Propanol, 1,2-Propandiol und gegebenenfalls einen oder mehrere aliphatische Alkohole anders als Propanol und 1,2-Propandiol, die eine Anzahl an Kohlenstoffatomen umfasst zwischen 1 und 4 aufweisen, wobei die Zusammensetzung umfasst:
- von 60,0% bis 99,7% eines Kohlenwasserstoffgemisches, das einem Benzingemisch entspricht,
- von 0,2 bis 20% Propanol,
- von 0,1 bis 10% 1,2-Propandiol und
- gegebenenfalls von 0% bis 20% eines oder mehrerer aliphatischer Alkohole anders als Propanol und 1,2-Propandiol, die eine Anzahl an Kohlenstoffatomen umfasst zwischen 1 und 4 aufweisen,
wobei das Propanol und 1,2-Propandiol jeweils in solchen Mengen vorliegen, dass ihr Volumenverhältnis zwischen 2 und 50 umfasst ist, und wobei das Benzingemisch durch einen Endpunkt gekennzeichnet ist, der 250°C nicht überschreitet, und eine Dichte zwischen 720 und 775 kg/m³ aufweist.

2. Zusammensetzung nach Anspruch 1, umfassend:
• von 80% bis 94% eines Kohlenwasserstoffgemisches, das einem Benzingemisch entspricht,
• von 1% bis 15% Propanol,
• von 0,5% bis 5% 1,2-Propandiol und
• gegebenenfalls von 0% bis 10% eines oder mehrerer aliphatischer Alkohole anders als Propanol und 1,2-Propandiol, die eine Anzahl an Kohlenstoffatomen umfasst zwischen 1 und 4 aufweisen,
wobei das Propanol und 1,2-Propandiol jeweils in solchen Mengen vorliegen, dass ihr Volumenverhältnis zwischen 3 und 10 umfasst ist.

3. Zusammensetzung nach Anspruch 1 oder 2, wobei der aliphatische Alkohol anders als Propanol und 1,2-Propandiol aus Ethanol, Methanol oder einem Gemisch der vorgenannten ausgewählt ist.

4. Zusammensetzung nach einem der vorhergehenden Ansprüche, umfassend Methanol in einer Menge von 0,1 Vol.-% bis 5 Vol.-%, bevorzugt von 1 Vol.-% bis 3 Vol.-%, weiter bevorzugt von 1,5 Vol.-% bis 2 Vol.-%, bezogen auf das Gesamtvolumen der Zusammensetzung.

5. Zusammensetzung nach einem der vorhergehenden Ansprüche, wobei das Propanol aus 0 bis 10% 2-Propanol zusammengesetzt ist, wobei der verbleibende Teil 1-Propanol darstellt.

6. Zusammensetzung nach einem der vorhergehenden Ansprüche, wobei das Propanol aus 10 bis 30% 1-Propanol zusammengesetzt ist, wobei der verbleibende Teil 2-Propanol darstellt.

7. Zusammensetzung nach einem der vorhergehenden Ansprüche, wobei das Propanol aus 10,1% bis 50% 2-Propanol zusammengesetzt ist, wobei der verbleibende Teil 1-Propanol darstellt.

8. Zusammensetzung nach einem der vorhergehenden Ansprüche, wobei das Benzingemisch durch einen Endpunkt gekennzeichnet ist, der 210°C nicht überschreitet.

9. Zusammensetzung nach einem der vorhergehenden Ansprüche, umfassend Wasser in einer Menge von weniger als 10000 Gewichts-ppm, bevorzugt weniger als 5000 Gewichts-ppm, weiter bevorzugt in dem Bereich von 50 bis 1000 Gewichts-ppm, bezogen auf das Gesamtgewicht der Zusammensetzung.

10. Verfahren zur Darstellung einer Kraftstoffzusammensetzung nach einem der vorhergehenden Ansprüche von 1 bis 9, ausgehend von Glycerin, bevorzugt biologischen Ursprungs, und einem Benzingemisch, bevorzugt bleifreies Benzin und mit einem niedrigen Schwefelgehalt, wobei das Verfahren die folgenden Schritte umfasst:
a) Hydrierung von Glycerin, um ein erstes Gemisch zu erhalten, das Wasser, Propanol und 1,2-Propandiol umfasst, wobei das Propanol und 1,2-Propandiol in solchen Anteilen vorliegen, dass ihr Volumenverhältnis zwischen 2 und 50, bevorzugt zwischen 3 und 10, umfasst ist;
b) Abtrennung von Wasser und mindestens einem Teil nicht umgesetzten Glycerins aus dem ersten Gemisch, abgeleitet in Schritt (a), um ein zweites Gemisch zu erhalten, das Propanol und 1,2-Propandiol umfasst; und
c) Hinzufügen des zweiten Gemisches zu einem Benzingemisch, bevorzugt fossilen Ursprungs, in Mengen umfasst zwischen 0,2 Vol.-% und 40 Vol.-%, bevorzugt zwischen 1 und 30 Vol.-%, bezogen auf das Volumen des resultierenden Gemisches, um eine Kraftstoffzusammensetzung zu erhalten.

11. Verfahren nach Anspruch 10, wobei in dem Schritt (a) die Umsetzung von Glycerin mindestens 50%, weiter bevorzugt von 55% bis 99%, noch weiter bevorzugt von 60% bis 90%, beträgt.

12. Verfahren nach einem der vorhergehenden Ansprüche 10 oder 11, wobei das zweite Gemisch, erhalten in Schritt (b), im Wesentlichen aus Propanol, 1,2-Propandiol und bis zu maximal 10 Vol.-% von C1-C3-Alkoholen anders als Glycerin zusammengesetzt ist.

13. Verfahren nach einem der vorhergehenden Ansprüche 10 bis 12, wobei das Propanol in dem zweiten Gemisch, erhalten in Schritt (b), von 0 bis 10% 2-Propanol umfasst, wobei der verbleibende Teil 1-Propanol darstellt.

14. Verfahren nach einem der vorhergehenden Ansprüche 10 bis 13, wobei der Hydrierungsschritt (a) in der Gegenwart eines Hydrierungskatalysators bei einer Temperatur umfasst zwischen 100°C und 400°C unter Wasserstoffdruck umfasst zwischen 0,1 MPa und 10 MPa durchgeführt wird.

15. Verfahren nach einem der vorhergehenden Ansprüche 10 bis 14, wobei in dem Schritt (c) Methanol auch zu dem Benzingemisch in einer Menge umfasst zwischen 0,1 Vol.-% und 5 Vol.-%, weiter bevorzugt zwischen 1 Vol.-% und 3 Vol.-%, bezogen auf das Gesamtvolumen der Zusammensetzung, hinzugefügt wird.

## Revendications

1. Composition utile en tant que carburant pour des moteurs à essence comprenant un mélange d'hydrocarbures correspondant à un mélange d'essence, du propanol, du propane-1,2-diol et facultativement un ou plusieurs alcools aliphatiques, autres que le propanol et le propane-1,2-diol, ayant un nombre d'atomes de carbone compris entre 1 et 4, ladite composition comprenant :
- de 60,0 % à 99,7 % d'un mélange d'hydrocarbures correspondant à un mélange d'essence,
- de 0,2 à 20 % de propanol,
- de 0,1 à 10 % de propane-1,2-diol, et
- facultativement de 0 % à 20 % d'un ou plusieurs alcools aliphatiques, autres que le propanol et le propane-1,2-diol, ayant un nombre d'atomes de carbone compris entre 1 et 4
dans laquelle lesdits propanol et propane-1,2-diol sont respectivement dans des quantités telles que leur rapport en volume est compris entre 2 et 50, et dans laquelle ledit mélange d'essence est **caractérisé par** un point final ne dépassant pas 250 °C et a une masse volumique comprise entre 720 et 775 kg/m³.

2. Composition selon la revendication 1, comprenant :
• de 80 % à 94 % d'un mélange d'hydrocarbures correspondant à un mélange d'essence,
• de 1 % à 15 % de propanol,
• de 0,5 % à 5 % de propane-1,2-diol, et
• facultativement de 0 % à 10 % d'un ou plusieurs alcools aliphatiques, autres que le propanol et le propane-1,2-diol, ayant un nombre d'atomes de carbone compris entre 1 et 4,
dans laquelle lesdits propanol et propane-1,2-diol sont respectivement dans des quantités telles que leur rapport en volume est compris entre 3 et 10.

3. Composition selon la revendication 1 ou 2, dans laquelle ledit alcool aliphatique, autre que le propanol et le propane-1,2-diol, est choisi parmi l'éthanol, le méthanol ou un mélange de ceux-ci.

4. Composition selon l'une quelconque des revendications précédentes, comprenant du méthanol en une quantité de 0,1 % à 5 %, de préférence de 1 % à 3 %, plus préférablement de 1,5 % à 2 % en volume, par rapport au volume total de la composition.

5. Composition selon l'une quelconque des revendications précédentes, dans laquelle ledit propanol est constitué de 0 à 10 % de propan-2-ol, la partie restante étant du propan-1-ol.

6. Composition selon l'une quelconque des revendications précédentes, dans laquelle ledit propanol est constitué de 10 à 30 % de propan-1-ol, la partie restante étant du propan-2-ol.

7. Composition selon l'une quelconque des revendications précédentes, dans laquelle ledit propanol est constitué de 10,1 % à 50 % de propan-2-ol, la partie restante étant du propan-1-ol.

8. Composition selon l'une quelconque des revendications précédentes, dans laquelle ledit mélange d'essence est **caractérisé par** un point final ne dépassant pas 210 °C.

9. Composition selon l'une quelconque des revendications précédentes, comprenant de l'eau en une quantité inférieure à 10 000 ppm, de préférence inférieure à 5 000 ppm, plus préférablement dans la plage de 50 à 1 000 ppm, en poids par rapport au poids total de la composition.

10. Procédé de préparation d'une composition de carburant selon l'une quelconque des revendications précédentes 1 à 9, à partir de glycérine, de préférence d'origine biologique, et d'un mélange d'essence, de préférence d'essence sans plomb et avec une faible teneur en soufre, ledit procédé comprenant les étapes suivantes :
a) hydrogénation de glycérine pour obtenir un premier mélange comprenant de l'eau, du propanol et du propane-1,2-diol, dans lequel lesdits propanol et propane-1,2-diol sont dans des proportions telles que leur rapport en volume est compris entre 2 et 50, de préférence entre 3 et 10 ;
b) séparation de l'eau et d'au moins une partie de la glycérine n'ayant pas réagi à partir dudit premier mélange dérivé dans l'étape (a), pour obtenir un deuxième mélange comprenant du propanol et du propane-1,2-diol ; et
c) ajout dudit deuxième mélange à un mélange d'essence, de préférence d'origine fossile, dans des quantités comprises entre 0,2 % et 40 %, de préférence entre 1 et 30 % en volume, par rapport au volume du mélange résultant, pour obtenir une composition de carburant.

11. Procédé selon la revendication 10, dans lequel, dans ladite étape (a), la conversion de glycérine est d'au moins 50 %, plus préférablement de 55 % à 99 %, encore plus préférablement de 60 % à 90 %.

12. Procédé selon l'une quelconque des revendications précédentes 10 ou 11, dans lequel ledit deuxième mélange obtenu dans l'étape (b) est sensiblement constitué de propanol, de propane-1,2-diol et jusqu'à un maximum de 10 % en volume d'alcools en C1-C3 autres que la glycérine.

13. Procédé selon l'une quelconque des revendications précédentes 10 à 12, dans lequel ledit propanol dans le deuxième mélange obtenu dans l'étape (b) comprend de 0 à 10 % de propan-2-ol, la partie restante étant du propan-1-ol.

14. Procédé selon l'une quelconque des revendications précédentes 10 à 13, dans lequel ladite étape d'hydrogénation (a) est conduite en présence d'un catalyseur d'hydrogénation à une température comprise entre 100 °C et 400 °C, sous une pression d'hydrogène comprise entre 0,1 MPa et 10 MPa.

15. Procédé selon l'une quelconque des revendications précédentes 10 à 14, dans lequel, dans ladite étape (c), du méthanol est en outre ajouté au mélange d'essence en une quantité comprise entre 0,1 % et 5 % en volume, plus préférablement entre 1 % et 3 % en volume, par rapport au volume total de la composition.
